# DEMANDE DE BREVET EUROPEEN

(11) **EP 2 939 743 A1**
(43) Date de publication de la demande: **04.11.2015**
(21) Numéro de dépôt: 15305336.8
(22) Date de dépôt: 04.03.2015
(51) Int. Cl.: B01J 31/18, B01J 31/22, B01J 31/24

(54) **Nouveaux complexes à base de nickel et leur utilisation dans un procede de transformations des olefines**

(30) Priorité: 28.04.2014 FR 1453817
(71) Demandeur: IFP Énergies nouvelles, 92852 Rueil-Malmaison Cedex (FR); Universiteit van Amsterdam, 1000 GG Amsterdam (NL)
(72) Inventeur: Boulens, Pierre, 69006 Lyon (FR); Breuil, Pierre-Alain, 69006 LYON (FR); Reek, Joost, NL-3817 BK Amersfoort (NL); Olivier-Bourbigou, Helene, 69230 SAINT GENIS-LAVAL (FR)
(74) Mandataire: Ruis, Alain

(57) **Abrégé**

L'invention décrit un nouveau type de complexe à base de nickel et sa méthode de préparation. L'invention concerne également l'utilisation dudit complexe dans un procédé de transformation des oléfines.

## Description

La présente invention concerne une nouvelle famille de complexes à base de nickel, et leur méthode de préparation. L'invention concerne également l'utilisation desdits complexes comme catalyseurs de réactions de transformations chimiques.

### Art antérieur :

Il est connu de préparer des complexes à base de métaux de transition pour leur application dans divers domaines de la chimie, notamment dans le domaine des transformations catalytiques tels que l'hydroformylation, l'hydrogénation, le couplage croisé, l'oligomérisation des oléfines...

La préparation de tels complexes passe par le choix du métal et des ligands adaptés. Parmi ces ligands, les ligands bidentes représentent une classe importante de ligands utilisés dans la préparation de catalyseurs à base de métaux de transition pour divers types de transformations chimiques catalytiques.

Le document EP 2 220 099 B1 décrit un système de complexes de coordination comprenant des ligands multidentes ayant la formule: R₁-SO₂-NH-P(XR₂)₂; ou R₁-SO₂-N=PH(XR₂)₂, ou R₁-SO(OH)=NP(XR₂)₂, dans lesquels X est indépendamment O, S, NH, ou une liaison; dans lequel R₁ et R₂ sont indépendamment choisis parmi un groupe alkyle substitué ou non et un groupe aryle, dans lequel au moins un équivalent de ligand est complexé à un équivalent d'un métal choisi parmi le rhodium, l'iridium, le platine, le palladium et les lanthanides. EP 2 220 099 B1 indique que le système de complexe de coordination peut être utilisé en tant que catalyseur pour l'hydroformylation, l'hydrogénation, l'hydrogénation, la polymérisation, l'isomérisation...

La demanderesse dans ses recherches a mis au point une nouvelle famille de complexes à base de nickel et leur méthode de préparation. Il a été constaté de manière surprenante que de tels complexes présentent des propriétés catalytiques intéressantes. En particulier, ces catalyseurs présentent une bonne activité dans l'oligomerisation des oléfines, plus précisément dans la dimérisation de l'éthylène en butène-1. Ces complexes présentent également une bonne sélectivité dans la dimérisation de l'éthylène en butène-1.

Un objectif de l'invention est de fournir une nouvelle famille de complexes à base de nickel. Un autre objectif de l'invention est de proposer un nouveau système catalytique comprenant lesdits complexes pour des réactions de transformations chimiques, en particulier pour l'oligomerisation des oléfines.

### Description détaillée de l'invention

### Complexes de nickel

Le complexe selon l'invention est un complexe à base de nickel répondant à la formule (I) 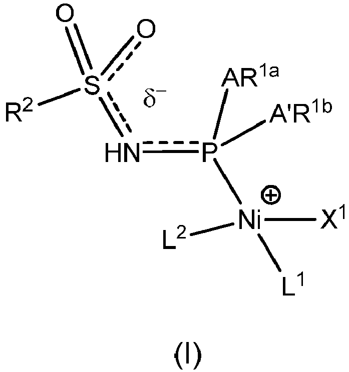 dans lequel
- les atomes P, N, S, O constituent un fragment de ligand,
- A et A', identiques ou différents, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R¹, représentés sur la formule par R^{1a} et R^{1b}, avec R^{1a} et R^{1b} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R² est choisi parmi les groupements alkyles cycliques ou non, substitués ou non et contenant ou non des hétéroéléments, les groupements aromatiques substitués ou non et contenant ou non des hétéroéléments,
- L¹ et L² identiques ou différents, représentent une base de Lewis,
- X¹ est un atome de carbone lié à ou faisant partie d'au moins un groupement alkyle cyclique ou non, insaturé ou non, substitué ou non et contenant ou non des hétéroéléments, un groupement aromatique substitué ou non et contenant ou non des hétéroéléments,
- L¹, L² et X¹ sont tels que le degré d'oxydation du nickel est respecté, et δ ⁻ représente la délocalisation de la charge négative sur le fragment de ligand constitué par les atomes P, N, S et O.

Au sens de la présente invention, on entend par « alkyle » une chaîne hydrocarbonée linéaire ou ramifiée ayant de 1 à 15 atomes de carbone, de préférence de 1 à 10. Des groupes alkyles préférés sont avantageusement choisis parmi les groupes méthyle, éthyle, propyle, isopropyle, butyle, isobutyle et tertio-butyle. Ces groupements alkyles peuvent être substitués par des hétéroéléments ou des groupes contenant des hétéroéléments tels qu'un halogénure, un alcoxy. On entend par un substituant « alcoxy », un groupe alkyl-O- dans lequel le terme alkyle a la signification donnée ci-dessus. Des exemples préférés de substituants alcoxy sont les groupes méthoxy ou éthoxy.

Par « alkyle cyclique », on entend on entend un groupe hydrocarboné monocyclique ayant un nombre de carbone supérieur à 3, de préférence entre 4 et 24, de manière plus préférée entre 6 et 12, de préférence un groupe cyclopentyle, cyclohexyle, cyclooctyle ou cyclododécyle, ou polycyclique (bi- ou tricyclique) ayant un nombre de carbone supérieur à 3, de préférence entre 4 et 18, tels que par exemple les groupes adamantyle ou norbornyle.

Par « alkyle insaturé linéaire » ou « alkyle insaturé cyclique », on entend un alkyle linéaire ou cyclique possédant au moins une insaturation, le terme alkyle et alkyle cyclique ayant la signification donnée ci-dessus.

Par « aromatique », on entend un groupe mono- ou polycyclique aromatique, de préférence mono- ou bicyclique, ayant un nombre d'atomes de carbone entre 5 et 20. Lorsque le groupe est polycyclique, c'est-à-dire qu'il comprend plus d'un noyau cyclique, les noyaux cycliques peuvent avantageusement être condensés deux à deux ou rattachés deux à deux par des liaisons σ. Le groupe aromatique selon l'invention peut contenir un hétéroélément tel que l'azote, l'oxygène ou le soufre.

Le terme ligand selon la présente invention est indifféremment utilisé pour signifier une ou plusieurs des formes limites du ligand répondant à la formule 1a), 1b) et/ou 1c): 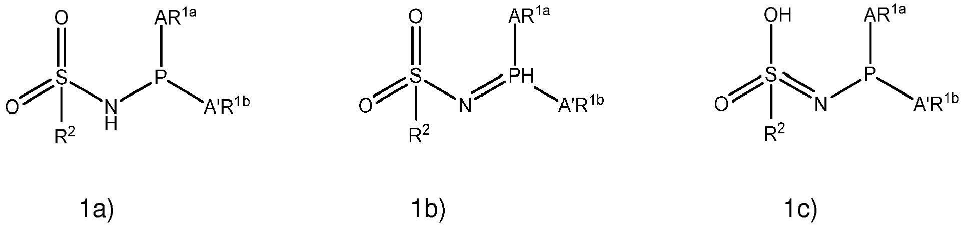 dans laquelle
- A et A', identiques ou différents, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R¹, représentés sur la formule par R^{1a} et R^{1b}, avec R^{1a} et R^{1b} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R² est choisi parmi les groupements alkyles cycliques ou non, substitués ou non et contenant ou non des hétéroéléments, les groupements aromatiques substitués ou non et contenant ou non des hétéroéléments.

Les deux groupements R¹ (R^{1a} et R^{1b}) peuvent être identiques ou différents entre eux. Ces deux groupements R^{1a} et R^{1b} peuvent également être liés entre eux. Dans un tel cas, les deux groupements R¹ peuvent correspondre à des groupements tels que le bis-phényle, le bis-naphthyle.

Les ligands selon l'invention peuvent être préparés par une réaction de condensation d'un sulfonamide, par exemple le para-*n*-butylphényl-sulfonamide et d'un halogénure de phosphine, tel que Ph₂PCl, en présence d'une base de Brönsted telle que la triéthylamine par exemple, dans un solvant. En solution, ces ligands peuvent (co)exister sous les trois formes 1a), 1b) ou 1c) décrites ci-dessus.

L¹ et L² identiques ou différents, liés ou non entre eux, représentent une base de Lewis. Au sens de la présente invention, on entend par « base de Lewis », toute entité chimique dont un constituant possède un doublet ou plus d'électrons libres ou non liants. Les bases de Lewis selon l'invention correspondent en particulier à tout ligand comprenant un atome d'oxygène, d'azote ou de phosphore possédant un doublet d'électrons libres ou non liants, ou une double liaison π capable de former avec le nickel une coordination de type η².

Le groupement L² du complexe de formule (I) selon l'invention peut représenter une phosphine de type P(A¹R'^{1a})(A'¹R'^{1b})(A"¹R'^{1c}) ou une phosphinamine de type (R'^{1a}A¹)(R'^{1b}A'¹)P-NH(R'²) ou (R'^{1a}A¹)(R'^{1b}A'¹)P-NH-S(O)₂R'²), dans lesquelles :
- A¹, A'¹ et A"¹, identiques ou différents entre eux, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R'¹ soit R^{'1a}, R^{'1b} et R^{'1c} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R'² est choisi parmi les groupements alkyles cycliques ou non, substitués ou non et contenant ou non des hétéroéléments, les groupements aromatiques substitués ou non et contenant ou non des hétéroéléments.

Lorsque le groupement X¹ est un atome de carbone lié à ou faisant partie d'au moins un groupement alkyle insaturé linéaire ou cyclique, X¹ et L¹ sont avantageusement liés de sorte à former un fragment allyle d'un alkyle linéaire ou cyclique permettant une liaison de type π nickel-allyle.

Selon l'invention, les groupements R¹ soit R^{1a} et R^{1b}, identiques ou différents, liés ou non entre eux, et les groupements R'¹ soit R^{'1a}, R^{'1b} et R^{'1c}, identiques ou différents, liés ou non entre eux, sont indépendamment choisis parmi les groupements alkyles comprenant 1 à 15 atomes de carbones, les groupements aromatique comprenant 5 à 20 atomes de carbone; substitués ou non, contenant des hétéroéléments ou non.

De préférence, les groupements R¹, soit R^{1a} et R^{1b} étant identiques ou différents, liés ou non entre eux, et les groupements R'¹, soit R^{'1a}, R^{'1b} et R^{'1c} étant identiques ou différents, liés ou non entre eux, sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant ou non des hétéroéléments; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-di(trifluorométhyl)phényle, benzyle, naphthyle, bisnaphthyle, pyridyle, bisphényle, furanyle, thiophényle, substitués ou non, contenant des hétéroéléments ou non. Dans le cas où les groupements R¹ soit R^{1a} et R^{1b} identiques ou différents sont liés entre eux, ces groupements peuvent correspondre à des groupements tels que le bis-phényle, le bis-naphthyle. Dans le cas où les groupements R'¹ identiques ou différents sont liés entre eux, ces groupements peuvent correspondre à des groupements tels que le bis-phényle, le bis-naphthyle.

Selon l'invention, les groupements R² et les groupements R'², identiques ou différents, sont indépendamment choisis parmi les groupements alkyles comprenant 1 à 15 atomes de carbones, les groupements aromatiques comprenant 5 à 20 atomes de carbone; substitués ou non, contenant des hétéroéléments ou non.

De préférence, les groupements R² et les groupements R'² , identiques ou différents, sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant des hétéroéléments ou non ; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-bis(trifluorométhyl)phényle, benzyle, naphthyle, bisnaphthyle, pyridyle, bisphényle, furanyle, thiophényle, substitués ou non, contenant des hétéroéléments ou non.

De préférence le groupement R³ est soit un atome d'hydrogène, soit un groupement alkyle.

Le complexe selon l'invention peut être préparé par la mise en contact d'au moins un ligand comprenant le fragment de ligand constitué par les atomes P, N, S et O répondant à la formule (1a), (1b) ou (1c) tel que défini selon l'invention, avec au moins un précurseur de nickel de degré d'oxydation (0), un précurseur du groupement X¹, un précurseur du groupement L¹ et optionnellement un précurseur du groupement base de Lewis L². La présence du groupement base de Lewis L² est par exemple optionnelle lorsqu'un deuxième équivalent du ligand est mis en oeuvre. Dans ce cas le ligand selon l'invention joue le rôle d'une base de Lewis. Avantageusement, les précurseurs des groupements X¹ et L¹ peuvent provenir du précurseur de nickel (0). C'est le cas par exemple, lorsque les précurseurs des groupements X¹ et L¹ forment entre eux un alkyle insaturé linéaire ou cyclique. De préférence, ledit alkyle insaturé linéaire ou cyclique est un diène.

Le complexe selon l'invention peut être préparé par la mise en contact d'au moins un ligand comprenant le fragment de ligand constitué par les atomes P, N, S et O répondant à la formule (1a), (1b) ou (1c) tel que défini selon l'invention, avec au moins un précurseur de nickel de degré d'oxydation (+II), en présence d'un précurseur du groupement X¹, d'un précurseur du groupement L¹, d'un agent réducteur et optionnellement d'un précurseur du groupement base de Lewis L². La présence du groupement base de Lewis L² est par exemple optionnelle lorsqu'un deuxième équivalent du ligand est mis en oeuvre. Dans ce cas le ligand selon l'invention joue le rôle d'une base de Lewis.

Dans le cas où un précurseur de nickel de degré d'oxydation (+II) est utilisé en présence d'un agent réducteur, on peut utiliser tout agent conduisant à la réduction du nickel connu de l'homme du métier. L'agent réducteur peut être choisi parmi NaBH₄, LiAlH₄, AlEt₃, Na, K, KC₈ et le dihydrogène.

La température de préparation du complexe selon l'invention peut varier entre -80°C et 130°C.

La préparation du complexe selon l'invention peut être réalisée en présence ou non de solvant. De préférence, la préparation est réalisée en présence d'un solvant. Le solvant de préparation peut être choisi parmi les solvants organiques et en particulier parmi les éthers, les alcools, les solvants chlorés et les hydrocarbures saturés, insaturés, aromatiques ou non, cycliques ou non. De préférence, le solvant est choisi parmi l'hexane, le cyclohexane, le méthylecyclohexane, l'heptane, le butane ou l'isobutane, les monooléfines ou dioléfines comportant de préférence 4 à 20 atomes de carbone, le cycloocta-1,5-diène, le benzène, le toluène, l'ortho-xylène, le mésitylène, l'éthylbenzène, le dichlorométhane, le chlorobenzène, le méthanol, l'éthanol, purs ou en mélange, et les liquides ioniques. Dans le cas où le solvant est un liquide ionique, il est avantageusement choisi parmi les liquides ioniques décrits dans les brevets US 6,951,831 B2 et FR 2895406 B1.

Le complexe selon l'invention comprend un nickel de degré d'oxydation (+I) ou (+II), de préférence un nickel de degré d'oxydation (+II). Les complexes selon l'invention peuvent également former des agrégats multi-nucléaires.

Lorsque le précurseur de nickel est de degré d'oxydation (0), ce dernier peut être choisi parmi le nickel(0) bis(cycloocta-1,5-diène), le nickel(0) bis(cycloocta-1,3-diène), le nickel(0) bis(cyclooctatétraène), le nickel(0) bis(cycloocta-1,3,7-triène), le bis(o-tolylphosphito)nickel(0)(éthylène), le nickel(0) tétrakis (triphénylphosphite), le nickel(0) tetrakis(triphenylphosphine), et le nickel (0) bis(éthylène), pris seul ou en mélange. Lesdits précurseurs de nickel peuvent éventuellement être complexés à des bases de Lewis.

Lorsque le précurseur de nickel est de degré d'oxydation (+II), ce dernier peut être choisi parmi le chlorure de nickel(II), le chlorure de nickel(II)(diméthoxyéthane), le bromure de nickel(II), le bromure de nickel(II)(diméthoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II) tel que par exemple le 2-éthylhexanoate, les phénates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de allylnickel(II), le bromure de allylnickel(II), le dimère du chlorure de methallylnickel(II), l'hexafluorophosphate de allylnickel(II), l'hexafluorophosphate de methallylnickel(II), le biscyclopentadienyle de nickel(II), le bisallyl de nickel(II) et le bisméthallyl nickel(II); sous leur forme hydratée ou non, pris seul ou en mélange. Lesdits précurseurs de nickel peuvent éventuellement être complexés à des bases de Lewis.

Nous présentons ci-dessous quelques exemples de complexes de formule (I) selon l'invention ainsi que les conditions opératoires au moyen desquelles ils ont été obtenus. Ces exemples sont à titre illustratif et ne sont en aucun cas limitatifs de la portée de l'invention.

Dans l'exemple du schéma 1, l'addition de 2 équivalents du ligand **L1,** à un équivalent de nickel(0) bis(cycloocta-1,5-diène) (Ni(COD)₂) dans du 1,5-cyclooctadiene conduit au complexe de formule (I) : **C1.** La préparation de ce complexe est réalisée à une température comprise entre -40°C et la température ambiante (TA) pendant 16 heures. Le complexe **C1** est caractérisé en RMN ³¹P par un large singulet à 54 ppm.

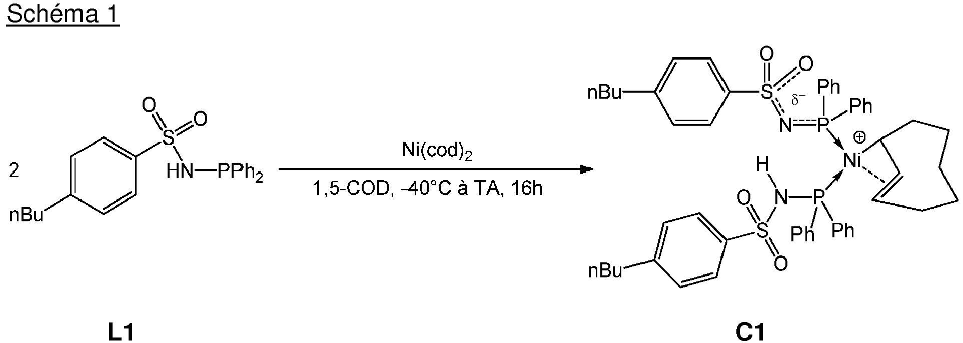

Un autre type de complexe de formule (I) selon l'invention est préparé par addition d'un équivalent du ligand **L2** ou **L3** et d'un équivalent de N-(diphenylphosphino)isopropylamine ((iPr)NHPPh₂) ou de N-(diphenylphosphino)-n-propylamine ((nPr)NHPPh₂) à un équivalent de nickel(0) bis(cycloocta-1,5-diène) dans du toluène ou du chlorobenzene. Les complexes **C2, C3, C4** et **C5** sont obtenus à une température comprise entre 0°C et 50°C au bou t d'une période allant de 1 à 16 heures (Schéma 2). Les complexes sont caractérisés en RMN ³¹P par deux doublets avec une constante de couplage de l'ordre de 30 Hz. 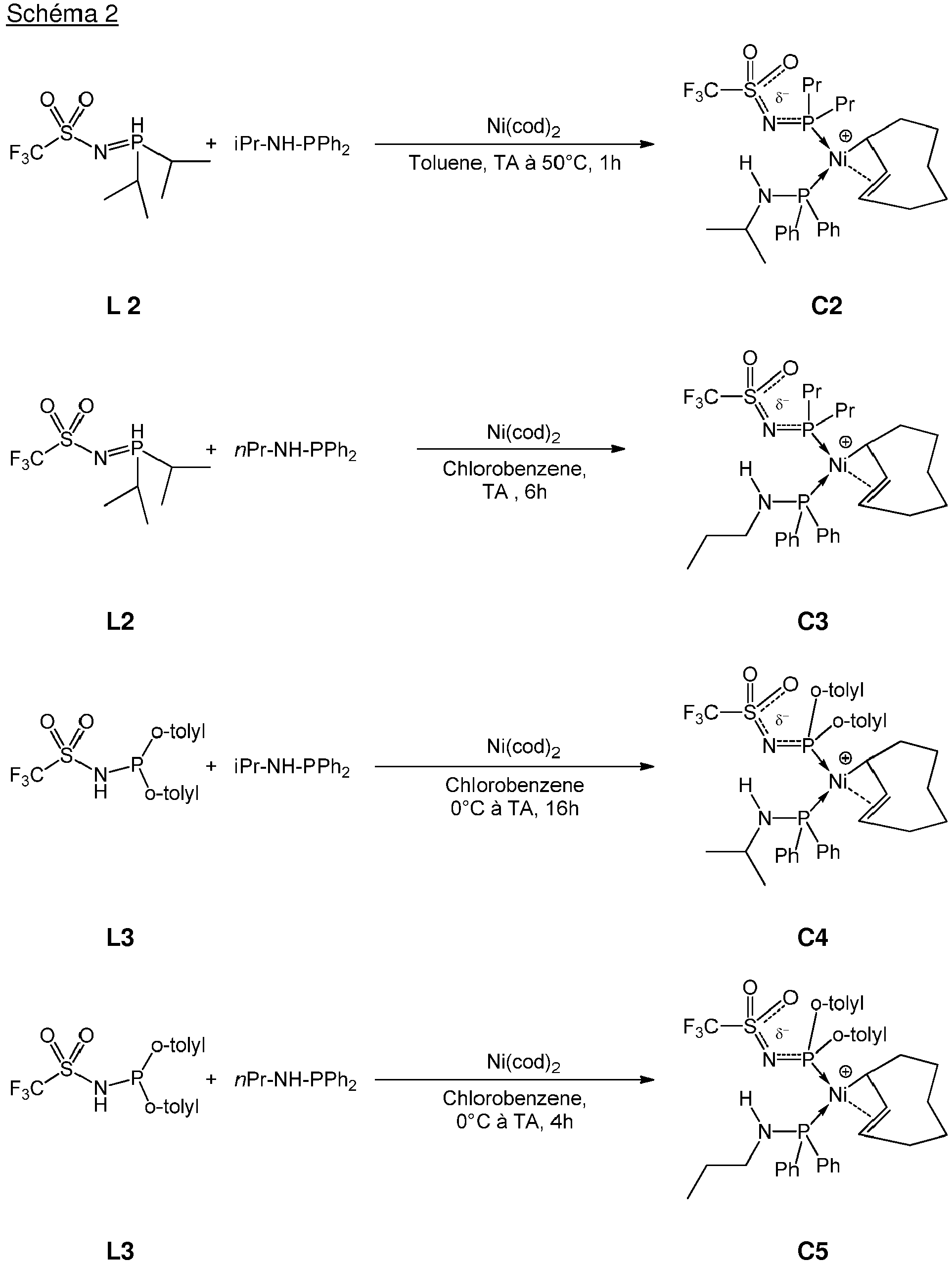 Le schéma 3 illustre par le groupement bisnaphtol, deux complexes de formule (I) dans lesquels les substituants R^{1a} et R^{1b} sont liés entre eux, A = O et dans lesquels le groupement R² est un groupement 4-n-butyl phényle (C6) ou un groupement -CF₃ (C7). Les complexes C6 et C7 sont préparés par addition d'un équivalent du ligand L4 ou L5 (ici représentés avec un équivalent de Et₃N) respectivement et d'un équivalent de triméthylphosphine à un équivalent de nickel(0) bis(cycloocta-1,5-diène) dans du chlorobenzène. Les complexes sont obtenus au bout de 3 heures à température ambiante (TA).

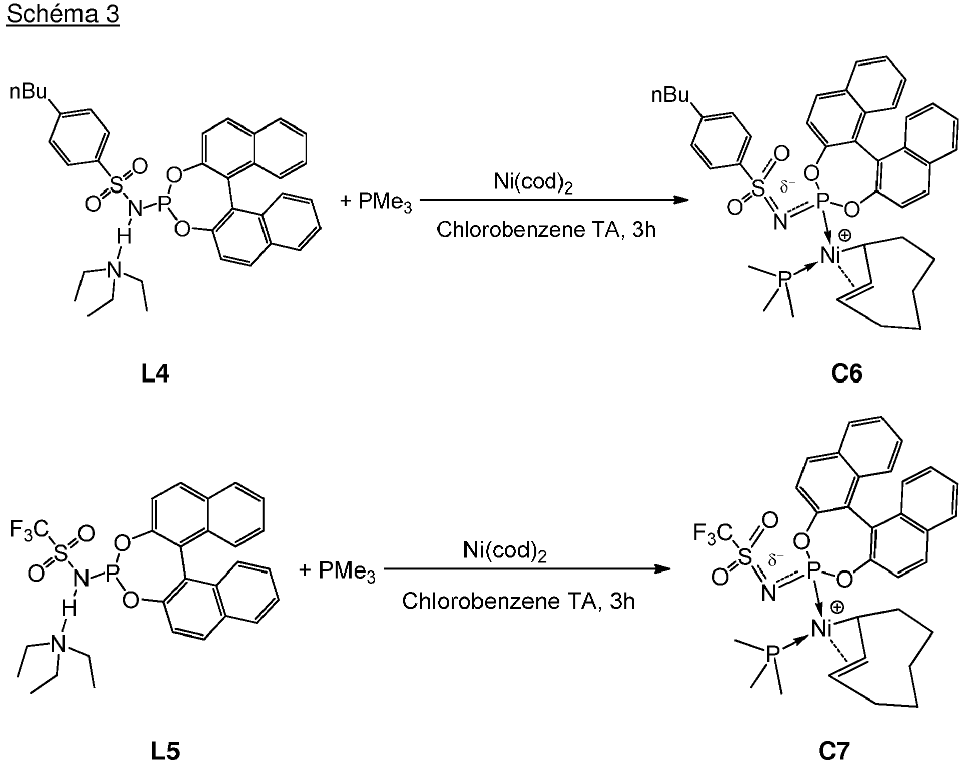

Des complexes similaires C8, **C9** et C10, possédant un ligand triméthylphosphine, sont obtenus dans les mêmes conditions avec les ligands respectifs L1, L2 et L3 (schéma 4). Ces complexes sont caractérisés en RMN ³¹P par deux doublets avec une constante de couplage de l'ordre de 30 Hz.

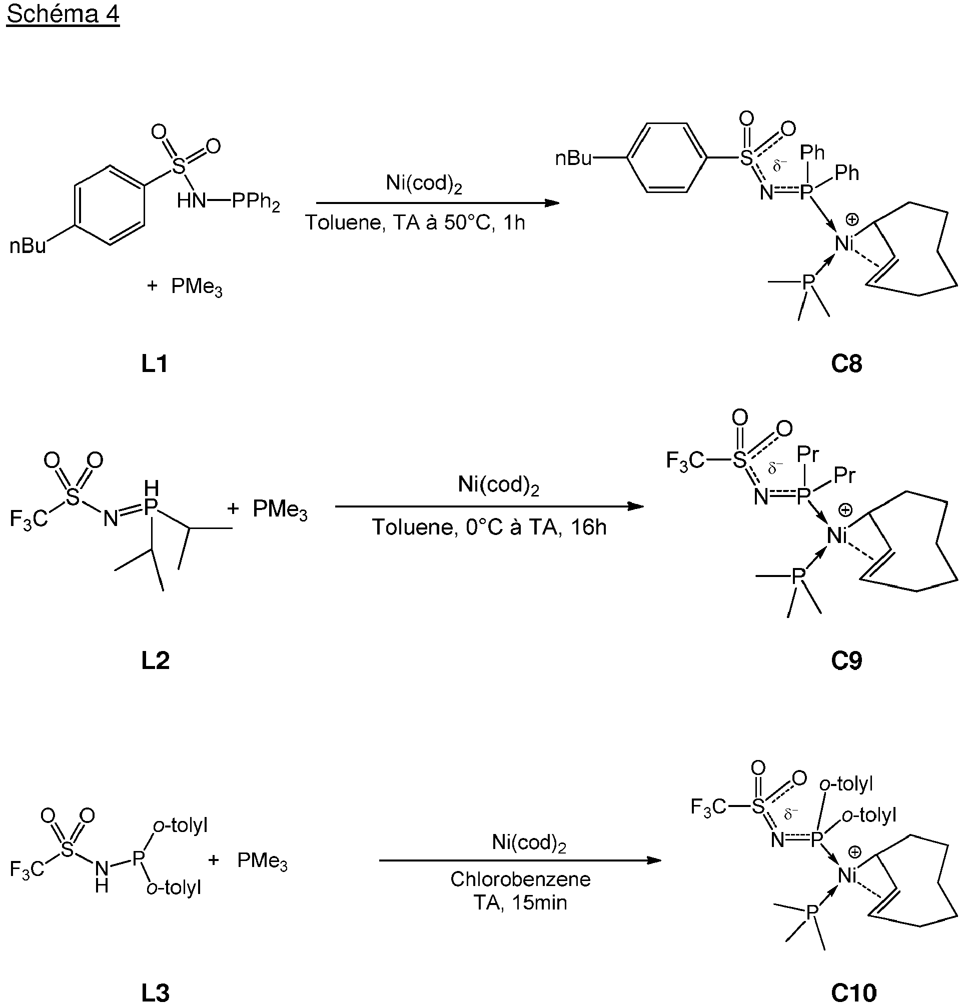

D'une manière préférée, les complexes selon l'invention répondent aux formules suivantes : 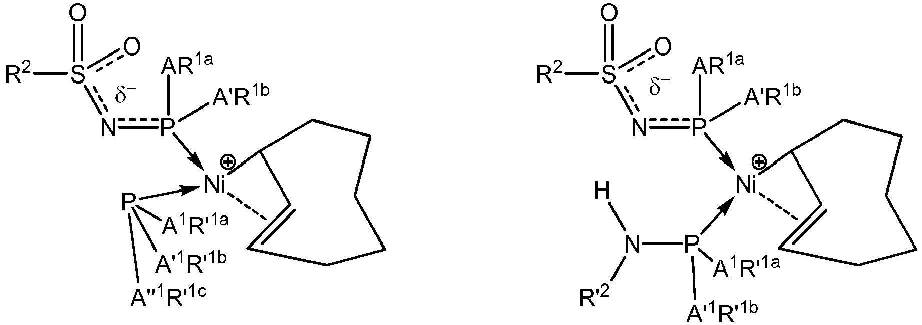 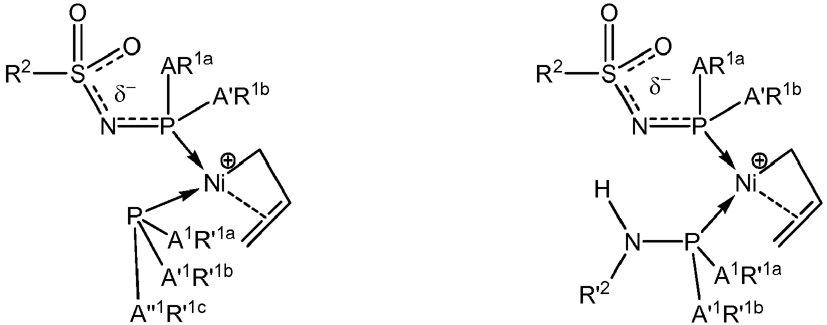 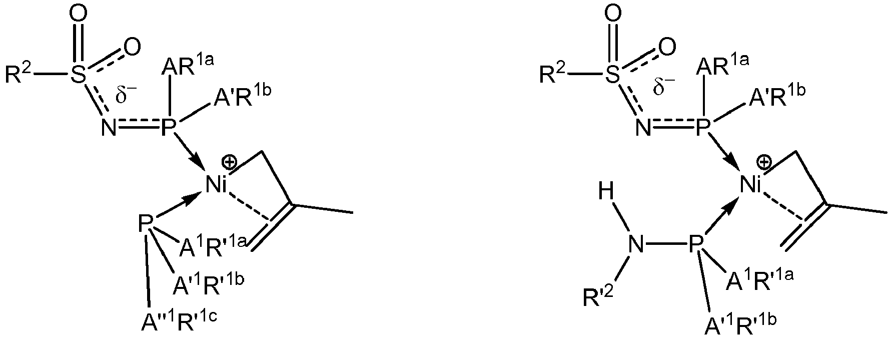 dans lesquels le nickel est de degré d'oxydation (+II), δ⁻ représente la délocalisation de la charge négative sur le fragment de ligand constitué par les atomes P, N, S et O et A, A', A¹, A'¹, A"¹, R^{1a}, R^{1b}, R'^{1a}, R'^{1b}, R'^{1c}, R² et R'² répondent aux spécifications selon l'invention.

Une liste non exhaustive de ligands pouvant convenir à la préparation des complexes selon l'invention est représentée ci-dessous. Les ligands sont ici représentés sous leurs formes limites 1 a) et 1 b).

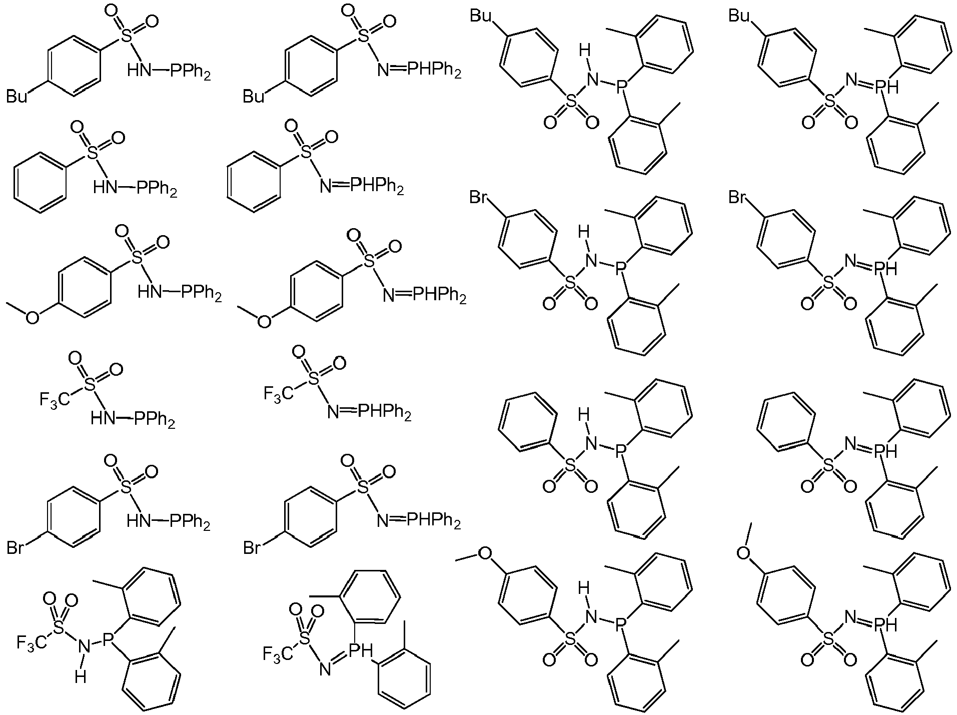

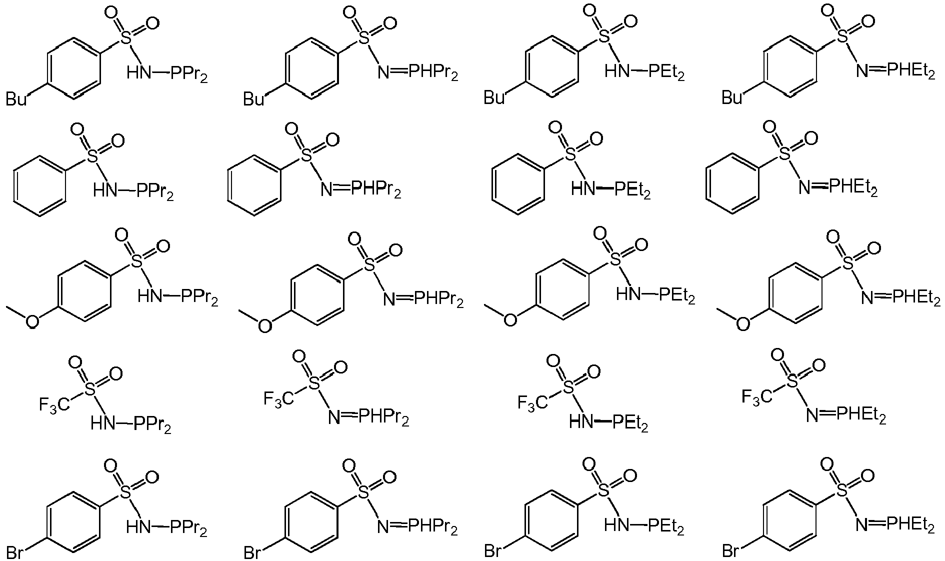

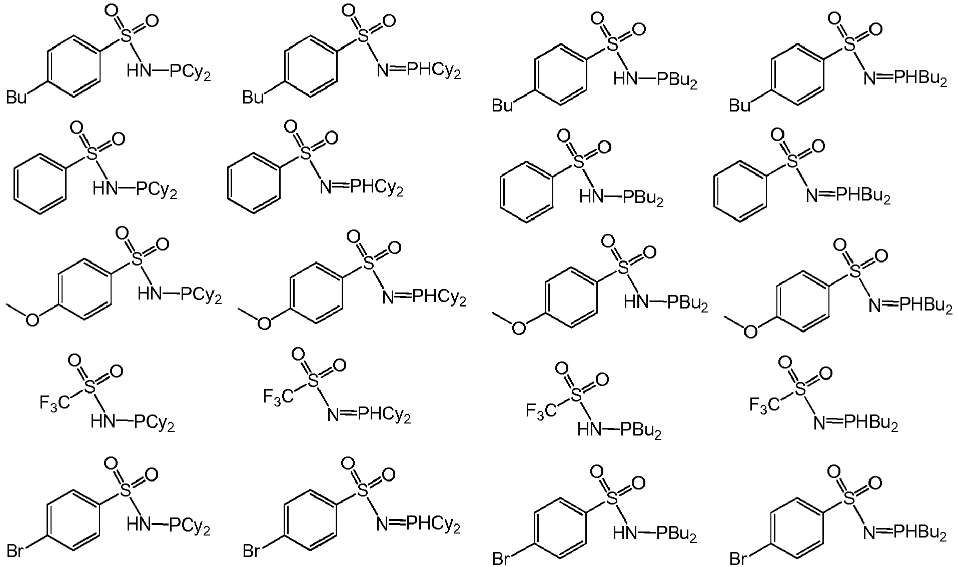

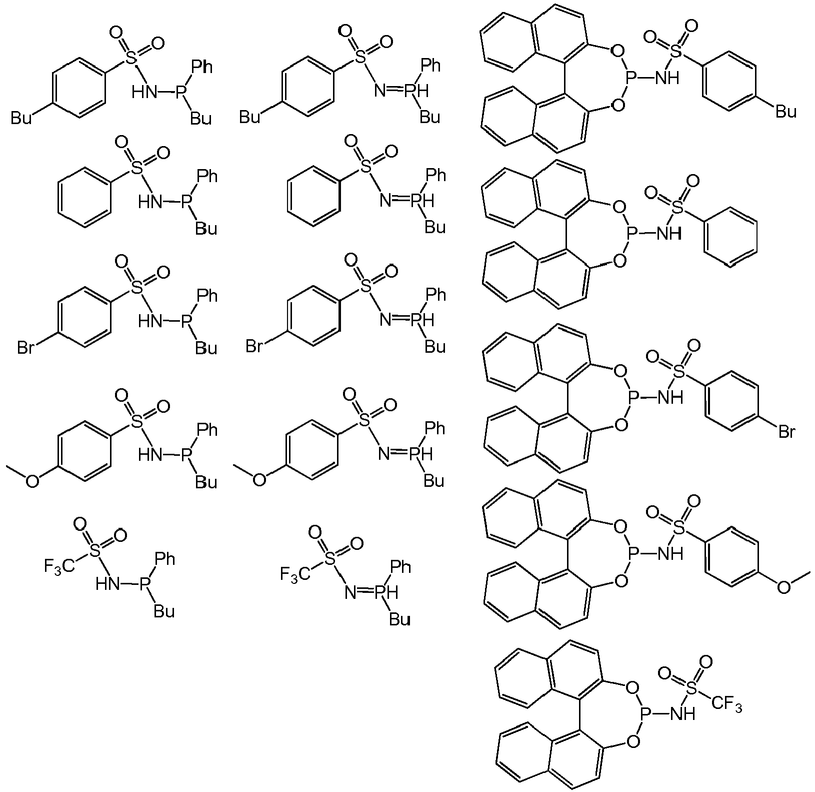

### Utilisation des complexes de formule (I) dans une réaction de transformation chimique

Les complexes à base de nickel de formule (I) selon l'invention peuvent être utilisés comme catalyseur dans une réaction de transformation chimique, telle que la réaction d'hydrogénation, d'hydroformylation, de couplage croisé ou d'oligomerisation des oléfines. En particulier, ces complexes sont utilisés dans un procédé d'oligomerisation d'une charge d'oléfines ayant avantageusement 2 à 10 atomes de carbone.

De préférence, le procédé d'oligomerisation est un procédé de dimérisation de l'éthylène en butene-1.

Le complexe de nickel de formule (I) selon l'invention peut être utilisé sous forme de composition catalytique, en mélange avec un composé appelé agent activateur. Ledit agent activateur est avantageusement choisi dans le groupe formé par les composés tris(hydrocarbyl)aluminium, les composés chlorés ou bromés d'hydrocarbylaluminium, les halogénures d'aluminium, les aluminoxanes, les composés organo-borés, les composés organiques susceptibles de donner ou de capter un proton, pris seuls ou en mélange.

Les tris(hydrocarbyl)aluminium, les composés chlorés ou bromés d'hydrocarbylaluminium et les halogénures d'aluminium répondent de préférence à la formule générale AlₓR_{y}W_{z} dans laquelle R représente un radical hydrocarboné monovalent contenant par exemple jusqu'à 12 atomes de carbone tel que alkyle, aryle, aralkyle, alkaryle ou cycloalkyle, W représente un atome d'halogène choisi par exemple parmi le chlore et le brome, W étant de préférence un atome de chlore, x prend une valeur de 1 à 2, y et z prennent une valeur de 0 à 3. Comme exemples de tels composés, on peut mentionner le sesquichlorure d'éthylaluminium (Et₃Al₂Cl₃), le dichlorure de méthylaluminium (MeAlCl₂), le dichlorure d'éthylaluminium (EtAlCl₂), le dichlorure d'isobutylaluminium (iBuAlCl₂), le chlorure de diéthylaluminium (Et₂AlCl), le triméthylaluminium, le tributylaluminium, le tri-n-octylaluminium et le triéthylaluminium (AlEt₃).

Dans le cas où ledit agent activateur est choisi parmi les aluminoxanes, ledit agent activateur est avantageusement choisi parmi le méthylaluminoxane (MAO), l'éthylaluminoxane et les méthylaluminoxanes modifiés (MMAO). Ces agents activateurs peuvent être utilisés seuls ou en mélange.

De préférence, ledit agent activateur C est choisi parmi le dichloroéthylaluminium (EtAlCl₂) et le méthylaluminoxane (MAO).

Dans le cas où ledit agent activateur est choisi parmi les composés organoborés, ledit agent activateur est de préférence choisi parmi les acides de Lewis de type tris(aryl)borane tels que le tris(perfluorophényl)borane, le tris(3,5-bis(trifluorométhyl)phényl)borane, le tris(2,3,4,6-tétrafluorophényl)borane, le tris(perfluoronaphtyl)borane, le tris(perfluobiphényl)borane et leurs dérivés et les (aryl)borates associés à un cation triphénylcarbénium ou à un cation ammonium trisubstitué tels que le triphénylcarbenium tétrakis(perfluorophényl)borate, le N,N-diméthylanilinium tétrakis(perfluorophényl)borate, le N,N-diéthylanilinium tétrakis(3,5-bis(trifluorométhyl)phényl)borate, le triphénylcarbenium tétrakis(3,5-bis(trifluorométhyl)phényl)borate.

Dans le cas où ledit agent activateur est choisi parmi les composés organiques susceptibles de donner un proton, ledit agent activateur est de préférence choisi parmi les acides de formule HY dans lequel Y représente un anion.

Dans le cas où ledit agent activateur est choisi parmi les composés organiques susceptibles de capter un proton, ledit agent activateur est de préférence choisi parmi les bases de Bronsted.

Le solvant du procédé d'oligomérisation peut être choisi parmi les solvants organiques et de préférence parmi les éthers, les alcools, les solvants chlorés et les hydrocarbures saturés, insaturés, aromatiques ou non, cycliques ou non. En particulier, ledit solvant est choisi parmi l'hexane, le cyclohexane, le méthylecyclohexane, l'heptane, le butane ou l'isobutane, les monooléfines ou dioléfines comportant de préférence 4 à 20 atomes de carbone, le cycloocta-1,5-diène, le benzène, le toluène, l'ortho-xylène, le mésitylène, l'éthylbenzène, le dichlorométhane, le chlorobenzène, le méthanol, l'éthanol, purs ou en mélange, et les liquides ioniques. Dans le cas où ledit solvant de réaction est un liquide ionique, il est avantageusement choisi parmi les liquides ioniques décrits dans les brevets US 6,951,831 B2 et FR 2895406 B1.

L'oligomérisation est définie comme la transformation d'une unité monomère en un composé ou mélange de composés de formule générale CₚH₂ₚ avec 4 ≤ p ≤ 80, de préférence avec 4 ≤ p ≤ 50, de manière préférée avec 4 ≤ p ≤ 26 et de manière plus préférée avec 4 ≤ p ≤ 14.

Les oléfines utilisées dans le procédé d'oligomérisation sont des oléfines comportant de 2 à 10 atomes de carbone. De préférence, lesdites oléfines sont choisies parmi l'éthylène, le propylène, les n-butènes et les n-pentènes, seules ou en mélange, pures ou diluées.

Dans le cas où lesdites oléfines sont diluées, lesdites oléfines sont diluées par un ou plusieurs alcane(s), tels qu'on les trouve dans des « coupes » issues des procédés de raffinage du pétrole, comme le craquage catalytique ou le craquage à la vapeur.

De manière préférée, l'oléfine utilisée dans le procédé d'oligomérisation est l'éthylène.

Lesdites oléfines peuvent venir de ressources non fossiles telles que la biomasse. Par exemple, les oléfines utilisées dans le procédé d'oligomérisation ou de dimérisation selon l'invention peuvent être produites à partir d'alcools, et en particulier par déshydratation des alcools.

La concentration du nickel dans la solution catalytique est avantageusement comprise entre 1.10⁻⁸ et 1 mol/L, et de préférence entre 1.10⁻⁶ et 1.10⁻² mol/L.

Le procédé d'oligomérisation opère avantageusement à une pression totale comprise entre la pression atmosphérique et 20 MPa, de préférence entre 0,1 et 8 MPa, et à une température comprise entre -40 et +250°C, de préférence entre -20°C et 150°C.

La chaleur engendrée par la réaction peut être éliminée par tous les moyens connus de l'homme du métier.

Le procédé d'oligomérisation peut être conduit en système fermé, en système semi-ouvert ou en continu, avec un ou plusieurs étages de réaction. Une vigoureuse agitation est avantageusement mise en oeuvre pour assurer un bon contact entre le ou les réactifs et le système catalytique.

Le procédé d'oligomérisation peut être mis en oeuvre en discontinu. Dans ce cas, un volume choisi de la solution comprenant le complexe selon l'invention est introduit dans un réacteur muni des dispositifs habituels d'agitation, de chauffage et de refroidissement.

Le procédé d'oligomerisation peut également être mis en oeuvre en continu. Dans ce cas, la solution comprenant le complexe selon l'invention est injectée en même temps que l'oléfine dans un réacteur agité par les moyens mécaniques classiques ou par une recirculation extérieure, et maintenue à la température souhaitée.

La composition catalytique est détruite par tout moyen habituel connu par l'homme du métier, puis les produits de la réaction ainsi que le solvant sont séparés, par exemple par distillation. L'oléfine qui n'a pas été transformée peut être recyclée dans le réacteur.

Le procédé selon l'invention peut être mis en oeuvre dans un réacteur à un ou plusieurs étages de réaction en série, la charge oléfinique et/ou la composition catalytique au préalable pré-conditionnée étant introduites en continu, soit dans le premier étage, soit dans le premier et un autre quelconque des étages. A la sortie du réacteur, la composition catalytique peut être désactivée, par exemple par injection d'ammoniac et/ou d'une solution aqueuse de soude et/ou d'une solution aqueuse d'acide sulfurique. Les oléfines non converties et les alcanes éventuellement présents dans la charge sont ensuite séparés des oligomères par distillation.

Les produits du présent procédé peuvent trouver une application par exemple comme composants de carburants pour automobiles, comme charges dans un procédé d'hydroformylation pour la synthèse d'aldéhydes et d'alcools, comme composants pour l'industrie chimique, pharmaceutique ou la parfumerie et/ou comme charges dans un procédé de métathèse pour la synthèse de propylène par exemple.

Les exemples suivants illustrent l'invention sans en limiter la portée. La notation Cy représente le groupement cyclohéxyle.

### Exemple 1:

### Synthèse du N-(diphenylphosphino)isopropylamine ((iPr)NHPPh2) selon la méthode décrite dans WO2008/077908.

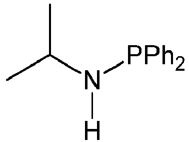

De l'isopropylamine (1.8 mL, 22.3 mmol, 2 eq.(équivalents) et de la triéthylamine (4,66 mL, 33.4 mmol, 3 eq.) sont placés dans un Schlenk avec 10 mL de tétrahydrofurane (THF). A ce mélange est ajoutée goutte-à-goutte de la chlorodiphenylphosphine (2 mL, 11.14 mmol, 1 eq.). Le mélange est agité pendant 10 minutes (min) à température ambiante et le précipité formé est filtré. Le filtrat est séché sous vide pour donner une huile incolore. La trituration de l'huile dans le pentane produit une poudre blanche. Cette poudre est rincée avec 2 x 10 mL de pentane. On isole 2,1 g de poudre blanche soit un rendement de 75%. Le produit pur est obtenu par distillation du solide à basse pression. Le produit est caractérisé par spectroscopie ³¹P RMN (C₆D₆), ¹H RMN (C₆D₆) et ¹³C NMR(C₆D₆).
³¹P NMR (C₆D₆): 34.94.

### Synthèse du N-(diphenylphosphino)-n-propylamine ((nPr)NHPPh₂) selon la méthode décrite dans WO2008/077908.

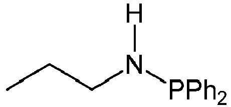

De la *n*-propylamine (3mL, 2.17g, 36.7 mmol, 3 eq.) est mélangée avec du THF (10 mL). A ce mélange est ajoutée goute à goute de la chlorodiphenylphosphine (2 mL, 2.46 g, 11.1 mmol, 1 eq.). Après 10 minutes d'agitation à température ambiante, le mélange est filtré et la phase liquide collectée. La phase liquide est évaporée sous pression réduite pour donner une huile jaune clair (isolé : 2.3 g, 85%).

### Synthèse des ligands L1, L2, L4, L5.

La synthèse des ligands **L1, L2, L4** et **L5** a été réalisée selon la méthode décrite dans la littérature : F. G. Terrade, Eur. J. Inorg. Chem. 2014, 1826-1835

### Synthèse du Ligand L3 metamorphos (o-tolyl)

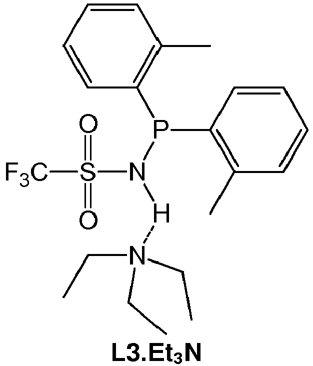

Du trifluorométhane sulfonamide (2.4 g, 16 mmol, 1 eq.) et de la triéthylamine (4.2 g, 40 mmol, 2.6 eq., 6 mL) sont solubilisés dans 30 mL de THF. Dans un deuxième Schlenk, la di(o-tolyl)chlorophosphine est solubilisée avec 10 mL de THF. La solution de chlorophosphine (4 g, 16 mmol, 1 eq.) dans 10 mL de THF est ajoutée goutte-à-goutte à la solution de sulfonamide pour produire un précipité blanc. Après 20 min, le mélange est filtré et le solide est rincé deux fois avec 10 mL de THF. La phase liquide est évaporée sous vide pour obtenir une huile incolore. 20 mL de diéthyléther sont rajoutés à ladite huile pour précipiter une poudre blanche. La poudre est rincée trois fois avec 5 mL de diéthyléther. Après séchage sous vide on obtient 2.52 g de poudre soit un rendement de 68%.
Le produit est caractérisé par spectroscopie ³¹P{¹H} RMN (C₆D₆), ³¹P RMN (C₆D₆), ¹H RMN (C₆D₆) et ¹³C NMR (C₆D₆).
³¹P NMR (C₆D₆) :15.77 (s, forme PH 12%) ; 22.62 (large s., forme NH-NEt₃, 88%).

### Synthèse du complexe C1

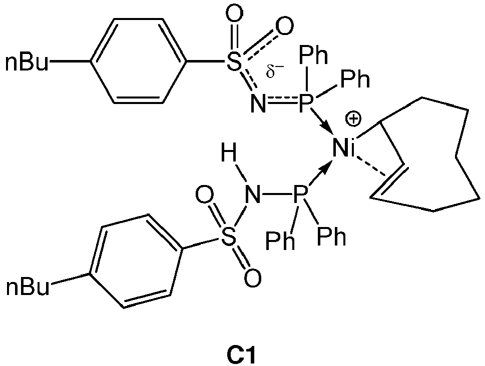

Le ligand L1 (4-nBu-Bz-SO₂-NH-PPh₂, 397 mg, 1 mmol, 2 eq.) et du Ni(COD)₂ sont solubilisés dans du 1,5-cyclooctadiene sec (10 mL) et le mélange est agité pendant 16 heures (h) jusqu'à disparition du signal du ligand par RMN du phosphore. Le solvant est évaporé pour donner une huile verte sombre. Cette huile est triturée avec du pentane plusieurs fois pour éliminer les traces de cyclooctadiène. L'huile est ensuite extraite par un mélange d'heptane/toluène (4 :1) et les fractions de solvant sont collectées et évaporées pour donner un solide orange sous forme de poudre. La poudre est lavée deux fois avec 10 mL de pentane pour obtenir une poudre orange (131 mg, rendement isolé : 27%).
³¹P NMR (C₆D₆): 54.06
Analyse Elémentaire trouvée (théorique): C: 64.17 (64.94); H: 6.64 (6.29); N: 3.15 (2,91).

### Synthèse du complexe C2

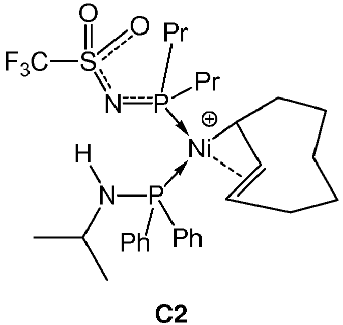

Le ligand **L2** (F₃C-SO₂-N=P(iPr)₂H, 395 mg, 1.5 mmol, 1 eq.), de la N-(diphenylphosphino)isopropylamine (365 mg, 1.5 mmol, 1 eq.) et du Ni(COD)₂ (413 mg, 1.5 mmol, 1 eq.) sont placés dans un Schlenk et solubilisés dans 30 mL de toluène auquel 4 gouttes de 1,5-cyclooctadiene sont ajoutées. La solution est ensuite chauffée à 50°C pendant 20 mn. Les solvants sont évaporés pour donner un solide huileux. Ce solide est trituré dans du pentane (10 mL) puis le pentane est retiré par seringue. L'opération est répétée une fois pour donner un solide puis lavé avec 3 x 10 mL de pentane. La poudre est dissoute dans le toluène et filtrée sur un disque seringue pour éliminer les résidus solides puis le filtrat ainsi obtenu est évaporé. La poudre obtenue est triturée dans le pentane puis lavée avec 2 x 10 mL de pentane pour enfin être séchée sous vide. Une poudre jaune est obtenue (rendement isolé : 574 mg, 57%).
³¹P RMN (C₆D₆): 85.76 (d, J=30.9 Hz); 60.22 (d, J=31.3 Hz)
Analyse Elémentaire trouvée (théorique): C 42.39 (42.54); H: 7.26 (7.14); N: 2.68 (2.76).

### Synthèse du complexe C3

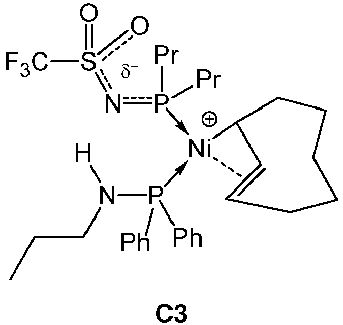

Le ligand **L2** (F₃C-SO₂-N=P(iPr)₂H, 265 mg, 1 mmol, 1 eq.), de la N-(diphenylphosphino)-n-propylamine (243 mg, 1 mmol, 1 eq.) et du Ni(COD)₂ (275 mg, 1 mmol, 1 eq.) sont placés dans un Schlenk et solubilisés dans 20 mL de chlorobenzène auquel 0.5 mL de 1,5-cyclooctadiene sont ajoutées. La solution est laissée agiter pendant 6h à température ambiante puis le solvant est évaporé sous pression réduite pour donner un solide jaune sombre. Ce solide est trituré puis lavé avec 2 x 10 mL de pentane. La poudre est alors séchée sous vide puis suspendue dans 5 mL de toluène. Le solvant est évaporé et l'opération est répétée une fois donnant une poudre très peu soluble dans le toluène. La poudre est ensuite dissoute dans 5 mL de dichlorométhane et la solution est filtrée sur un disque seringue 0.2 µm pour éliminer les insolubles. Le dichlorométhane est évaporé puis 5 mL de toluène sont ajoutés qui sont à leur tour évaporés sous vide. Une poudre jaune canari est obtenue (isolé : 325 mg, 47%).
³¹P NMR (CD₂Cl₂) : 64.54 (d, ²J_{PP}= 31 Hz) ; 92.16 (d, ²J_{PP}= 31 Hz).

### Synthèse du complexe C4

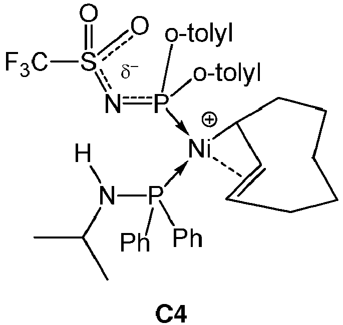

Le ligand **L3** (463 mg, 1 mmol, 1 eq.), de la N-(diphenylphosphino)isopropylamine (244 mg, 1 mmol, 1 eq.) et du Ni(COD)₂ (275 mg, 1 mmol, 1 eq.) sont solubilisés dans 20 mL of chlorobenzene à 0°C. Le mélange est agité à température ambiante pendant 16h pour donner une solution sombre. Le solvant est évaporé sous pression réduite pour conduire à une huile sombre. L'huile est triturée dans de l'éther diéthylique (10 mL) puis le solvant est retiré à l'aide d'une seringue. L'opération est répétée deux fois ce qui permet d'obtenir une poudre jaune qui est lavée avec de l'éther à 0°C jusqu' à ce que la phase d'éther ne s oit plus sombre. Le solide est alors séché sous vide pour donner le produit (190 mg isolé : 25% rendement).
³¹P NMR (C₆D₆): 52.13 (d, ²J_{PP}=23.1 Hz) ; 62.82 (d, ²J_{PP}=23.3 Hz) ;
Analyse Elémentaire trouvée (théorique):C: 51.62 (59.46); 5.25 (5.88); 3.22 (3.63).

### Synthèse du complexe C5

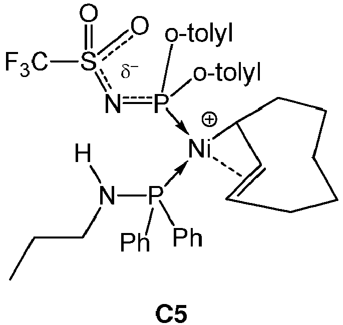

Le ligand **L3** (463 mg, 1 mmol, 1 eq.) de la N-(diphenylphosphino)-n-propylamine (243 mg, 1 mmol, 1 eq.) et du Ni(COD)₂ (275 mg, 1 mmol, 1 eq.) sont solubilisés dans 15 mL de chlorobenzene à 0°C. Le mélange est agité à température ambiante pendant 4h pour donner une solution marron sombre. Le solvant est évaporé sous pression réduite pour conduire à une huile sombre. L'huile est triturée et lavée avec du pentane (7 x 5 mL) ce qui conduit à la formation d'un solide jaune sombre qui est séché sous vide. Ce solide est ensuite lavé avec de l'éther diéthylique à 0°C (3 x 10 mL). L'éther est évaporé sous pression réduite puis la poudre est suspendue dans 5 mL de toluène qui est évaporé sous pression réduite. L'opération est répétée une fois et conduit à une poudre jaune clair (isolé 280 mg, 36%).
³¹P NMR (CD₂Cl₂): 52.33 (d, ²J_{PP}=24.7 Hz); 65.09 (d, ²J_{PP}=24.5 Hz).

### Synthèse du complexe C6

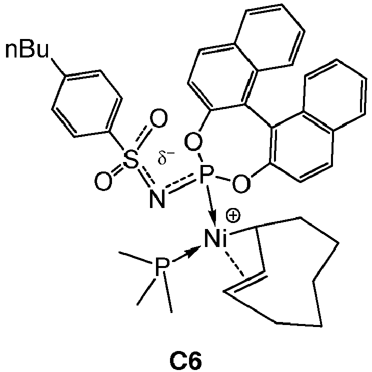

Le ligand **L4** (4-nBu-C₆H₄-SO₂-NH-P((R)-Binol) isolé avec un équivalent de triéthylamine, 53 mg, 0.1 mmol, 1 eq.) et du Ni(COD)₂ (28 mg, 0.1 mmol, 1 eq.) sont solubilisés dans du chlorobenzène (3 mL). A cette solution est ajouté une solution de triméthylphosphine (10% dans le toluène, 10µL, 0.1 mmol, 1 eq.). La solution devient rapidement marron et est agitée pendant 3 h à température ambiante. Le solvant est évaporé donnant un solide marron qui est trituré et lavé avec 3 x 5 mL de pentane. Le solide est séché donnant une poudre jaune : rendement isolé 35%. Deux diastéréoisomères (a) et (b) sont formés dans un ratio 1:1.
³¹P NMR (C₆D₆): (a) -13.4 (d, ²J_{PP-cis}=36 Hz) et 155.3 (d, ²J_{PP-cis}=36 Hz)
(b) -12.7 (d, ²J_{PP-cis}=39 Hz) et 156.2 (d, ²J_{PP-cis}=39 Hz).

### Synthèse du complexe C7

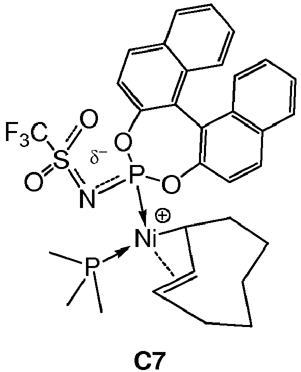

Le ligand **L5** (F₃C-NH-P((R)-Binol, isolé avec un équivalent de triéthylamine, 46 mg, 0.1 mmol, 1 eq.) et du Ni(COD)₂ (28 mg, 0.1 mmol, 1 eq.) sont solubilisés dans du chlorobenzène (3 mL). A cette solution est ajoutée une solution de triméthylphosphine (10% dans le toluène, 10µL, 0.1 mmol, 1 eq.). La solution devient rapidement marron et est agitée pendant 3h à température ambiante. Le solvant est évaporé donnant un solide marron qui est trituré et lavé avec 3 x 5 mL de pentane. Le solide est séché donnant une poudre jaune : rendement isolé 35%. Deux diastéréoisomères (a) et (b) sont formés dans un ratio 1:1.
³¹P NMR (C₆D₆): (a) -14.2 (d, ²J_{PP-cis}=33 Hz) et 157.6 (d, ²J_{PP-cis}=33 Hz)
(b) -13.6 (d, ²J_{PP-cis}=36 Hz) et 158.0 (d, ²J_{PP-cis}=36 Hz).

### Synthèse du complexe C8

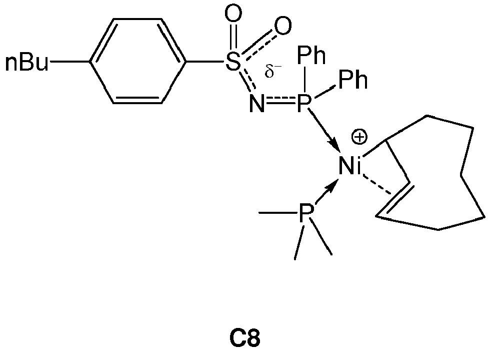

Le ligand **L1** (4-nBu-C₆H₄-SO₂-NH-PPh₂, 390 mg, 1 mmol, 1 eq) et de la triméthylphosphine (solution 1 M dans le toluène, 1 mL, 1 mmol, 1 eq.) sont placés dans un Schlenk et solubilisés dans 30 mL de toluène. Dans un autre Schlenk du Ni(COD)₂ (270 mg, 1 mmol, 1 eq) est solubilisé dans 20 mL de toluène. Les deux solutions sont refroidies dans un bain eau/glace et la solution contenant les phosphines est ajoutée par canule à la solution de nickel. Après addition, le mélange est laissé remonter à température ambiante et agité pendant 20 min. Il est finalement chauffé à 60°C pendant 1 h pour donner un liquide ma rron sombre. Le solvant est alors évaporé pour donner une poudre orange. Le solide est lavé avec du pentane pour donner une poudre orange : rendement isolé 36%.

³¹P NMR (C₆D₆): 60.25 (d, ²J_{PP-cis}=26.7 Hz); -0.11 (d, ²J_{PP-cis}=26.7 Hz)

Analyse Elémentaire trouvée (théorique): C 61.67 (61.69); H: 6.87 (7.08); N: 2.27 (2.19).

### Synthèse du complexe C9

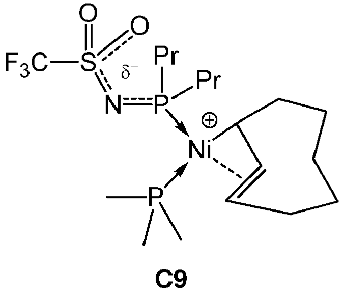

Le ligand **L2** (F₃C-SO₂-N=P(iPr)₂H, 106 mg, 0.4 mmol, 1 eq) et de la triméthylphosphine (solution 1 M dans le toluène, 0.5 mL, 0.5 mmol, 1.25 eq.) sont placés dans un Schlenk avec 30 mL de toluène. Dans un autre Schlenk du Ni(COD)₂ est solubilisé dans 20 mL de toluène. Les deux solutions sont refroidies dans un bain d'eau/glace et la solution contenant les phosphines est ajoutée à la solution de nickel. La solution est laissée sous agitation pendant 16h. Ensuite, le solvant est évaporé pour donner une poudre jaune qui est triturée puis lavée avec 3 x 10 mL de pentane puis séchée sous vide.

³¹P NMR (C₆D₆): 86.8 ppm (d, P(iPr)₂ *²J_{PP}* =31.05 Hz); -11.4 ppm (d, P(Me)₃ *²J_{PP}* =31.45 Hz).

### Synthèse du complexe C10

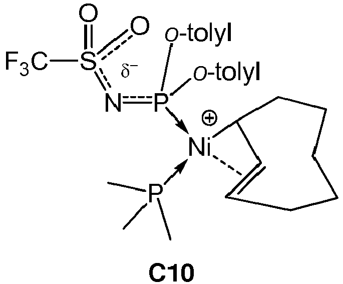

Le ligand **L3** (463 mg, 1 mmol, 1 eq.) et du Ni(COD)₂ (275 mg, 1 mmol, 1 eq.) sont solubilisés dans 20 mL of chlorobenzene. A cette solution est ajoutée de la triméthylphosphine (1 M dans le toluène, 1 mL, 1 mmol, 1 eq.). Le mélange est laissé sous agitation pendant 15 minutes conduisant à une solution sombre. Le solvant est alors éliminé sous pression réduite pour donner un solide sombre. Le solide est trituré avec 20 mL de pentane pour donner une poudre jaune et un solvant coloré en violet. Le solide est alors rincé avec du pentane jusqu'à ce que la couleur violette disparaisse. Le solide est séché sous vide pour donner une poudre jaune : rendement isolé 53%.

³¹P NMR (C₆D₆): -15.10 (d, *²J_{PP}*=25.8 Hz); 54.75 (d, *²J_{PP}*=25.8 Hz).

Analyse Elémentaire trouvée (théorique): C : 51.57 (51.68) ; H : 5.89 (6.00) ; N : 2.47 (2.33).

### Synthèse du complexe C11

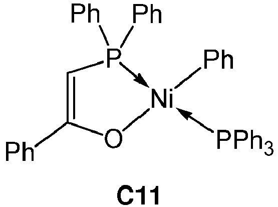

Le complexe de référence **C11** est synthétisé en accord avec la littérature (Organometallics 1986, 5, 2356-2359) et les caractérisations sont conformes au produit attendu.

### Exemple 2: oligomérisation de l'éthylène

La réaction d'oligomérisation de l'éthylène a été évaluée avec les complexes **C1, C2, C4, C8** et **C9** (10 µmoles) avec comme exemple comparatif le complexe **C11.** Les résultats obtenus sont reportés dans le tableau 1.

Le réacteur de 250 mL est séché sous vide à 130°C pendant 2 heures puis pressurisé avec 0,5 MPa d'éthylène. La température est abaissée à 20°C, puis la surpression d'éthylène est évacuée pour atteindre 0,1 MPa. Le solvant est ajouté (45 mL de toluène), et la consigne de température interne est fixée (40°C ou 80°C). Une fois la température interne stabilisée, le complexe est introduit (10 µmol dans 5 mL de toluène). Ensuite le réacteur est pressurisé avec de l'éthylène à 3 MPa. L'agitation (1000 tr/min) est mise en route (t=0). Après le temps de réaction défini, le mélange est refroidi à 30°C sous agitation, le réacteur est dépressurisé et les phases liquide et gaz sont analysées par chromatographie en phase gazeuse (GC).

La productivité (g_{oligo}/(g_{Ni}.h) est exprimée en masse d'oligomères produits (en gramme) par masse de nickel mis en oeuvre et par heure.

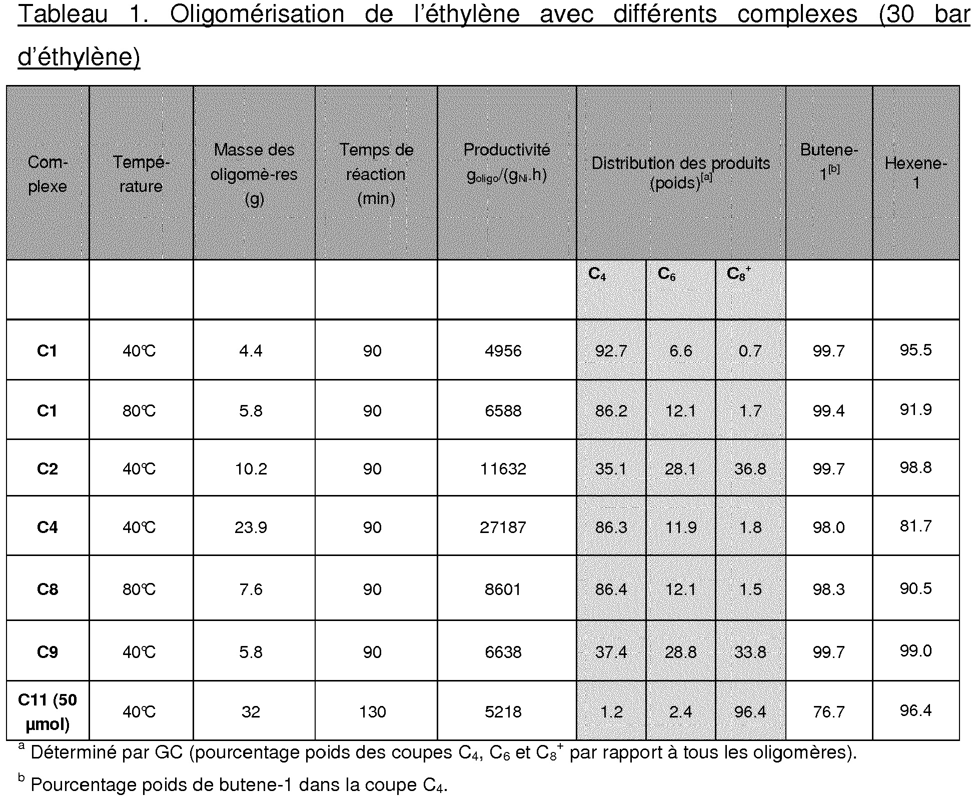

Les exemples ci-dessus démontrent que les complexes selon l'invention présentent une bonne activité pour l'oligomérisation de l'éthylène.

## Revendications

1. Complexe à base de nickel répondant à la formule (I) 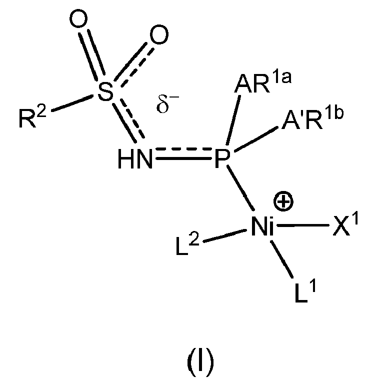 dans lequel
- les atomes P, N, S, O constituent un fragment de ligand,
- A et A', identiques ou différents, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R¹, représentés sur la formule par R^{1a} et R^{1b}, avec R^{1a} et R^{1b} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R² est choisi parmi les groupements alkyles cycliques ou non, substitués ou non et contenant ou non des hétéroéléments, les groupements aromatiques substitués ou non et contenant ou non des hétéroéléments,
- L¹ et L², identiques ou différents, représentent une base de Lewis,
- X¹ est un atome de carbone lié à ou faisant partie d'au moins un groupement alkyle cyclique ou non, insaturé ou non, substitué ou non et contenant ou non des hétéroéléments, un groupement aromatique substitué ou non et contenant ou non des hétéroéléments,
- L¹, L² et X¹ sont tels que le degré d'oxydation du nickel est respecté, et δ ⁻ représente la délocalisation de la charge négative sur le fragment de ligand constitué par les atomes P, N, S et O.

2. Complexe selon la revendication 1 dans lequel L² représente une phosphine de type P(A¹R'^{1a})(A'¹ R'^{1b})(A"¹ R'^{1c}) ou une phosphinamine de type (R'^{1a}A¹)(R'^{1b}A'¹)P-NH(R'²) ou (R'^{1a}A¹)(R'^{1b}A'¹)P-NH-S(O)₂(R'²), dans lesquelles :
- A¹, A'¹ et A"¹, identiques ou différents entre eux, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R'¹ soit R^{'1a}, R^{'1b} et R^{'1c} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R'² est choisi parmi les groupements alkyles cycliques ou non, substitués ou non et contenant ou non des hétéroéléments, les groupements aromatiques substitués ou non et contenant ou non des hétéroéléments.

3. Complexe selon la revendication 1 ou 2 dans lequel lorsque X¹ est un atome de carbone lié à ou faisant partie d'au moins un groupement alkyle insaturé linéaire ou cyclique, X¹ et L¹ étant liés de sorte à former un fragment allyle d'un alkyle linéaire ou cyclique permettant une liaison de type π nickel-allyle.

4. Complexe selon l'une des revendications 1 à 3 dans lequel les groupements R¹soit R^{1a} et R^{1b} étant identiques ou différents, liés ou non entre eux, et les groupements R'¹, soit R^{'1a}, R^{'1b} et R^{'1c} étant identiques ou différents, liés ou non entre eux, sont choisis indépendamment parmi les groupements alkyles comprenant 1 à 15 atomes de carbones, les groupements aromatiques comprenant 5 à 20 atomes de carbone; substitués ou non, contenant des hétéroéléments ou non.

5. Complexe selon la revendication 4 dans lequel les groupements R¹, soit R^{1a} et R^{1b} étant identiques ou différents, liés ou non entre eux, et les groupements R'¹, soit R^{'1a}, R^{'1b} et R^{'1c} étant identiques ou différents, liés ou non entre eux, sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant ou non des hétéroéléments; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-di(trifluorométhyl)phényle, benzyle, naphthyle, bisnaphthyle, pyridyle, bisphényle, furanyle, thiophényle, substitués ou non, contenant des hétéroéléments ou non.

6. Complexe selon l'une des revendications 1 à 5 dans lequel les groupements R² et les groupements R'², identiques ou différents, sont indépendamment choisis parmi les groupements alkyles comprenant 1 à 15 atomes de carbones, les groupements aromatiques comprenant 5 à 20 atomes de carbone; substitués ou non, contenant des hétéroéléments ou non.

7. Complexe selon la revendication 6 dans lequel les groupements R² et les groupements R'², identiques ou différents, sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant des hétéroéléments ou non ; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-bis(trifluorométhyl)phényle, benzyle, naphthyle, bisnaphthyle, pyridyle, bisphényle, furanyle, thiophényle, substitués ou non, contenant des hétéroéléments ou non.

8. Procédé de préparation d'un complexe selon l'une des revendications 1 à 7 comprenant la mise en contact d'au moins un ligand comprenant ledit fragment de ligand constitué par les atomes P, N, S et O, avec au moins un précurseur de nickel de degré d'oxydation (0), un précurseur du groupement X¹, un précurseur du groupement L¹ et optionnellement un précurseur du groupement base de Lewis L².

9. Procédé selon la revendication 8 dans lequel le précurseur de nickel est choisi parmi le nickel(0) bis(cycloocta-1,5-diène), le nickel(0) bis(cycloocta-1,3-diène), le nickel(0) bis(cyclooctatétraène), le nickel(0) bis(cycloocta-1,3,7-triène), le bis(o-tolylphosphito)nickel(0)(éthylène), le nickel(0) tétrakis (triphénylphosphite), le nickel(0) tetrakis(triphenylphosphine), et le nickel (0) bis(éthylène), pris seul ou en mélange.

10. Procédé de préparation d'un complexe selon l'une des revendications 1 à 7 comprenant la mise en contact d'au moins un ligand comprenant ledit fragment de ligand constitué par les atomes P, N, S et O, avec au moins un précurseur de nickel de degré d'oxydation (+II), en présence d'un précurseur du groupement X¹, d'un précurseur du groupement L¹, d'un agent réducteur et optionnellement d'un précurseur du groupement base de Lewis L².

11. Procédé selon la revendication 10 dans lequel le précurseur de nickel est choisi parmi le chlorure de nickel(II), le chlorure de nickel(II)(diméthoxyéthane), le bromure de nickel(II), le bromure de nickel(II)(diméthoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II) tel que par exemple le 2-éthylhexanoate, les phénates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de allylnickel(II), le bromure de allylnickel(II), le dimère du chlorure de methallylnickel(II), l'hexafluorophosphate de allylnickel(II), l'hexafluorophosphate de methallylnickel(II), le biscyclopentadienyle de nickel(II), le bisallyl de nickel(II) et le bisméthallyl nickel(II); sous leur forme hydratée ou non, pris seul ou en mélange.

12. Utilisation d'un complexe selon l'une des revendications 1 à 7 ou préparé selon l'une des revendications 8 à 11 en tant que catalyseur.

13. Procédé d'oligomérisation d'une charge d'oléfines comprenant la mise en contact de ladite charge avec un complexe selon l'une des revendications 1 à 7 ou préparé selon l'une des revendications 8 à 11, en présence ou pas de solvant.

14. Procédé selon la revendication 13 dans lequel le complexe est utilisé en mélange avec un composé choisi dans le groupe formé par les composés tris(hydrocarbyl)aluminium, les composés chlorés ou bromés d'hydrocarbylaluminium, les aluminoxanes, les composés organo-borés, les composés organiques susceptibles de donner ou capter un proton, pris seuls ou en mélange.

15. Procédé selon la revendication 13 ou 14 dans lequel la charge comprend des oléfines ayant un nombre d'atomes de carbone compris entre 2 et 10.

16. Procédé selon l'une des revendications 13 à 15 dans lequel la réaction est une réaction d'oligomérisation de l'éthylène.
